# EUROPÄISCHE PATENTSCHRIFT

(11) **EP 1 634 555 B1**
(45) Veröffentlichungstag und Bekanntmachung des Hinweises auf die Patenterteilung: **09.06.2010**
(21) Anmeldenummer: 05018284.9
(22) Anmeldetag: 23.08.2005
(51) Int. Cl.: A61F 13/00, A61F 13/06

(54) **Mit einem Polster versehene Gelenkbandage**
Joint bandage with pad
Bandage avec coussin

(30) Priorität: 23.08.2004 DE 102004040800
(43) Veröffentlichungstag der Anmeldung: 15.03.2006
(73) Patentinhaber: Bauerfeind AG, 07937 Zeulenroda (DE)
(72) Erfinder: Reinhardt, Holger, 47906 Kempen (DE)
(74) Vertreter: Bardehle, Heinz

(56) Entgegenhaltungen:
- EP-A- 0 262 638
- EP-A- 0 934 732
- DE-A1- 3 902 434
- DE-C2- 19 824 649
- DE-U1- 20 005 663
- US-A1- 2002 082 542

## Beschreibung

Die Erfindung bezieht sich auf eine aus elastischem Textilmaterial bestehende Gelenkbandage, die mit einem Polster versehen ist, das von einem Überzug aus gleichem oder ähnlichem Textilmaterial abgedeckt und mittels über das Polster überstehender Ränder an dem Textilmaterial der Bandage befestigt ist.

Eine derartige Bandage ist in der DE-PS 38 32 438 beschreiben und dargestellt. Bei derartigen Bandagen besteht das Bedürfnis, diese möglichst rutschfest an dem betreffenden Gelenk anzubringen.
Hierzu hat man bereits am Textilmaterial der Bandage über einen in der neutralen Zone zwischen der Streck- und der Beugezone angeordneten Streifen mit Noppen angebracht, die z. B. aus Silikon bestehen, das gegenüber der Haut eine rutschhemmende Wirkung aufweist (siehe DE 198 24 649 C2).
Eine ähnliche Gestaltung ist aus der DE 39 02 434 A1 bekannt, in der eine Stützbandage ohne Polster auf der mit der Haut in Berührung kommenden Innenseite ihres elastischen Textilmaterials mit Antirutschelementen aus Silikonmaterial versehen ist, wobei die Rutschelemente aus kleinen, flachen Quadern bestehen, die insoweit eine gewisse Ähnlichkeit mit Noppen besitzen.

Diese Gestaltungen erfordern für eine gute Rutschfestigkeit eine entsprechende Beschichtung des Textilmaterials der Bandage über einen größeren Bereich bzw. größere Länge, um der Bandage einen ausreichend festen Sitz zu geben.

Es ist darüber hinaus für eine aus der US-PS 6,520,926 B2 bekannt gewordene Kniegelenkbandage angegeben worden, die gesamte Innenseite der Bandage mit Noppen aus Kunststoff zu versehen. Eine derartige Gestaltung erschwert jedoch ganz erheblich das Überziehen der Bandage, z. B. über Fuß und Unterschenkel, was normalerweise mit beiden Händen geschieht, wobei in die Bandage an gegenüberliegenden Stellen mit den Händen eingegriffen und die Bandage dabei gedehnt wird, um sie über das Betreffende Glied überhaupt streifen zu können. Hierbei ergibt sich der für den Benutzer unangenehme Effekt, dass bei dieser Ausdehnung der Bandage Bereiche zwischen den Beiden Händen besonders an die Haut des betreffenden Gliedes angedrückt werden, so dass das Überstreifen der Bandage durch die Noppen aus rutschendem Material besonders erschwert wird.

Der Erfindung liegt die Aufgabe zugrunde, die eingangs erwähnte Gelenkbandage, die mit einem Polster versehen ist, so zu gestalten, dass unter Vermeidung einer Behinderung des Überziehens der Bandage mit ihr einen besonders hohe Rutschfestigkeit erzielbar ist. Erfindungsgemäß geschieht dies dadurch, dass der das Polster abdeckende Überzug auf seiner dem Gelenk zugewandten Seite mit Noppen aus rutschhemmendem Material versehen ist. der das Polster abdeckende Überzug auf seiner dem Gelenk zugewandten Seite mit Noppen aus rutschhemmendem Material versehen ist.

Bei der erfindungsgemäßen Gelenkbandage mit Polster wird für die notwendige Rutschhemmung der Bereich des Polsters ausgenutzt, da dieser aus der Bandage gewissermaßen hervorspringt und dadurch in seinem Bereich einen besonderen Druck auf die betreffenden Gelenkteile ausübt. Dieser Druck wird ausgenutzt, um im Bereich des Überzugs Noppen aus einem rutschhemmenden Material anzubringen, die aufgrund des besonderen Andrucks, der von dem Polster auf das betreffende Gelenk ausgeht, besonders stark haften und damit der Bandage praktisch absolute Rutschfestigkeit im Sinne einer Kraftschlüssigkeit geben. Als rutschhemmendes Material wird zweckmäßig Silikon verwendet. Dieses kann im flüssigen Zustand auf den Überzug aufgespritzt werden, an dem es dann unter klebender Verbindung mit dem Überzug aushärtet.

In den Figuren ist ein Ausführungsbeispiel der Erfindung dargestellt. Es zeigen:
- Figur 1: eine Kniegelenkbandage mit ringförmigem Polster, das die Kniescheibe umgibt;
- Figur 2: die gleiche Bandage in einer von innen nach außen gewendeten Lage, bei der also die Innenseite der Bandage gemäß Figur 1 nach außen hin sichtbar ist.
- Fig. 3: einen Schnitt III/III gemäss Figur 2

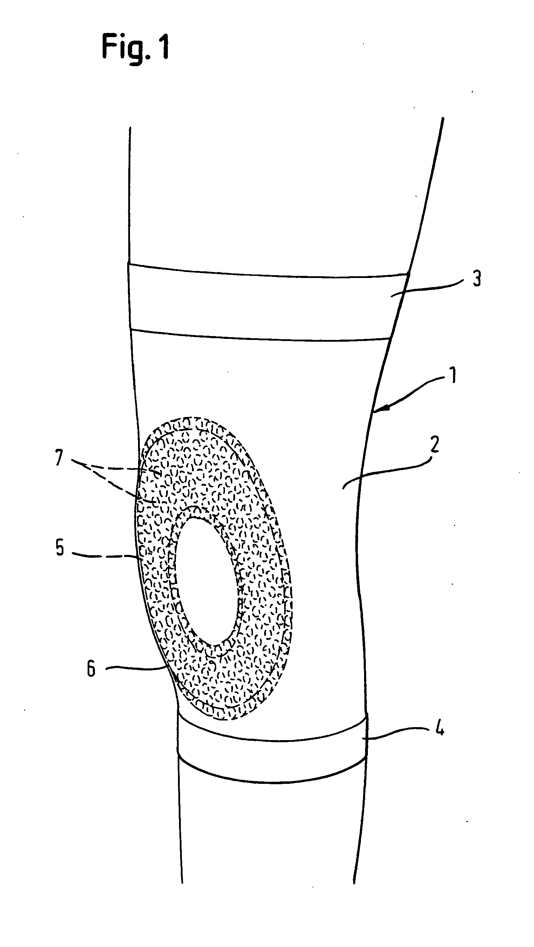

Für die Darstellung der erfindungsgemäßen Gelenkbandage wird die aus Figur 1 ersichtliche Kniegelenkbandage 1 gewählt. Es sei jedoch darauf hingewiesen, dass die erfindungsgemäße Bandage auch für jedes andere Gelenk Verwendung finden kann, bei dem ein in die Bandage integriertes Polster Verwendung findet, z.B. eine Ellenbogenbandage.

Die Bandage 1 besteht aus dem Strumpf 2 mit den beiden Abschlussrändern 3 und 4, die in üblicher Weise dafür sorgen, dass in ihrem Bereich die Bandage zu keiner Blutabsperrung führt. Im Bereich der Kniescheibe ist die Bandage mit dem ringförmigen Polster 5 versehen, das von dem Überzug 6 abgedeckt ist. Der Überzug 6 ist an dem Textilmaterial des Strumpfes 2 z.B. durch Kleben befestigt. Durch den Überzug 6 wird gewährleistet, dass das Polster 5 an der gewünschten richtigen Stelle der Bandage 1 an deren Strumpf 2 sicher befestigt ist.

Auf der dem Gelenk zugewandten Seite des Überzugs 6 (also in der Figur 1 gestrichelt, weil nicht sichtbar, dargestellt) ist dieser mit sich über praktisch die gesamte Fläche des Überzugs 6 erstreckenden Noppen 7 versehen, die kleine etwa halbkugelförmige Erhebungen bilden, aus Silikon bestehen und bei angelegter Bandage 1 am Kniegelenk liegende Haut des Gelenks drücken und damit eine hohe Rutschsicherheit gewährleisten, da sie aufgrund des nach innen vorspringenden Polsters mit besonderem Druck gegen die Haut des Gelenks gedrückt werden.

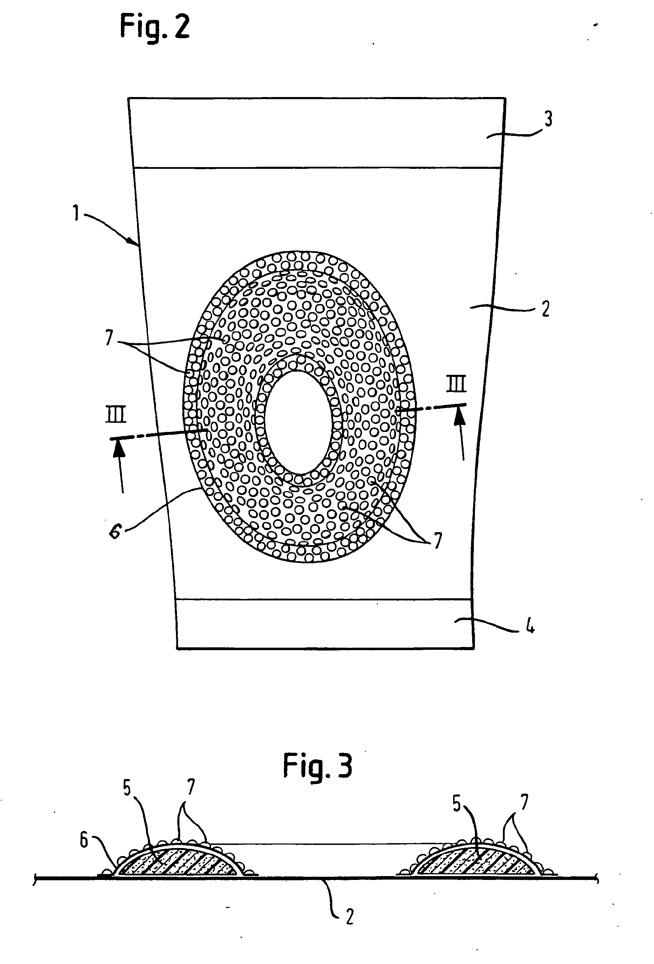

Figur 2 zeigt die gleiche Kniegelenkbandage, allerdings in sich umgedreht, so dass die bei normaler Benutzung nach innen gerichtete Seite nach außen gerichtet ist und damit für den Betrachter direkt die Beschichtung des Überzugs 6 mit den Noppen 7 zeigt.

In der Figur 3 ist die in Figur 2 dargestellte Bandage 1 gemäß dem Schnitt III-III wiedergegeben, und zwar nach Art einer Abwicklung, so dass der Strumpf 2 als gestreckte Linie erscheint. Wie ersichtlich ist das ringförmige Polster 5 von dem Überzug 6 abgedeckt, der die Noppen 7 trägt.

Aufgrund des besonders starken Andrückens der Noppen 7 gegen das Gelenk, insbesondere bei dessen Beugen, ergibt sich zusätzlich zu dem Antirutscheffekt der Noppen noch eine besonders intensive Massagewirkung auf den von den Noppen überdeckten Bereich des Gelenks, was in besonderen Fällen zusätzlich erwünscht ist.

## Patentansprüche

1. Aus elastischem Textilmaterial bestehende Gelenkbandage (1), die mit einem Polster (5) versehen ist, das von einem Überzug (6) aus gleichem oder ähnlichem Textilmaterial abgedeckt und mittels über das Polster (5) überstehender Ränder an dem Textilmaterial der Bandage (1) befestigt ist, **dadurch gekennzeichnet, dass** der das Polster (5) abdeckende Überzug (6) auf seiner dem Gelenk zugewandten Seite mit Noppen aus rutschhemmendem Material versehen ist.

2. Gelenkbandage nach Anspruch 1, **dadurch gekennzeichnet, dass** als rutschhemmendes Material Silikon verwendet wird.

## Claims

1. A joint bandage (1) consisting out of elastic fabric, which is provided with a pad (5), which is covered by an overlay (6) out of the same or similar fabric and is fixed to the fabric of the bandage (1) by means of edges extending beyond the pad (5), **characterised in that** the overlay (6) covering the pad (5) is provided on it's the joint facing side with pimpled (7) out of slide-inhibitive material.

2. Joint bandage according to claim 1, **characterised in that** silicone is used as slide-inhibitive material.

## Revendications

1. Bandage composé de tissu élastique (1) qui est pourvu d'un coussin (5), qui est couvert d'un revêtement (6) d'un tissu identique ou similaire et qui est fixé au moyen de rebords dépassant sur le coussin (5) sur le tissu du bandage (1), **caractérisé en ce que** le revêtement (6) couvrant le coussin (5) sur son côté tourné vers l'articulation est pourvu de picots en matériel antidérapant.

2. Bandage selon revendication 1, **caractérisé en ce que** de la silicone est utilisée en tant que matériel antidérapant.
